(19) Europäisches Patentamt
European Patent Office
Office européen des brevets

(11) **EP 1 225 445 A1**

(12) **EUROPÄISCHE PATENTANMELDUNG**

(43) Veröffentlichungstag:
**24.07.2002 Patentblatt 2002/30**

(51) Int Cl.7: **G01N 29/02**, G01N 29/22,
G01N 29/28, A61M 5/36,
A61M 1/36

(21) Anmeldenummer: **02000685.4**

(22) Anmeldetag: **11.01.2002**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **20.01.2001 DE 20101082 U**

(71) Anmelder: **B. BRAUN MELSUNGEN AG**
**34212 Melsungen (DE)**

(72) Erfinder:
• **Steger, Jürgen**
**34327 Körle (DE)**
• **Mosebach, Wolfgang**
**34323 Malsfeld (DE)**

(74) Vertreter: **Selting, Günther, Dipl.-Ing. et al**
**Patentanwälte**
**von Kreisler, Selting, Werner**
**Postfach 10 22 41**
**50462 Köln (DE)**

(54) **Ultraschallsensor zum Detektiern von Gasblasen**

(57) Der Ultraschallsensor enthält einen Ultraschallsender (11) und einen Ultraschallempfänger (12), zwischen denen sich eine Aufnahmevorrichtung (13) für einen Schlauch (14) befindet. Der Schlauch wird zwischen Stempeln (21,22) flachgedrückt und dadurch gegen starre konkave Formungsbereiche (15,16) gedrückt, wo eine spaltfreie Anlage an der Gehäusewand (17) erfolgt. Die Schallgeschwindigkeit ist im Gehäuse (10) etwa so groß wie im Schlauchmaterial (14). In der Flüssigkeit, die sich im Schlauch befindet, ist die Schallgeschwindigkeit wesentlich geringer. Parallele Schallwellen werden an dem Übergang zwischen Schlauchmaterial und Flüssigkeit so gebrochen, dass sie konvergieren. Durch die Bündelung der Schallenergie in den Schlauch und auf den Ultraschallempfänger (12) wird dann energiereiches Empfangssignal erzeugt. Bei Vorhandensein von Gasblasen in der Flüssigkeit verringert sich das Empfangssignal.

FIG.1

**Beschreibung**

Die Erfindung betrifft einen Ultraschallsensor zum Detektieren von Gasblasen in einem Schlauch, mit einem Ultraschallsender und einem Ultraschallempfänger, die auf entgegengesetzten Seiten einer Aufnahmevorrichtung für den Schlauch angeordnet sind.

Es ist bekannt, in der Medizintechnik Ultraschallsensoren zum Detektieren von Gasblasen einzusetzen. Die Ultraschallsensoren weisen einen Ultraschallsender und einen Ultraschallempfänger auf, die jeweils ein Piezoelement enthalten. Das Ultraschallsignal wird durch das Messvolumen hindurchgeleitet. Da Flüssigkeiten gute Schallleiter sind, Gase hingegen schlechte Schallleiter steht die Stärke der empfangenen Schallenergie in einem umgekehrten Verhältnis zu der im Messvolumen enthaltenen Gasmenge. Eine besondere Schwierigkeit ist die Ankopplung der Ultraschallenergie an den Schlauch, der das Messvolumen enthält. Bereits kleine Luftspalte zwischen der Schlauchaußenwand und dem Sensor führen zu einer Totalreflektion.

In DE 35 30 747 A1 ist ein Ultraschallsensor beschrieben, bei dem die Aufnahmevorrichtung, in die der Schlauch eingelegt wird, zwei Membranen aufweist, welche jeweils eine mit Flüssigkeit gefüllte Kammer begrenzen. Die beiden Kammern sind durch ein Gelenk miteinander verbunden. Beim Schließen der Vorrichtung werden die Membranen von dem Flüssigkeitsdruck vollflächig gegen die Außenfläche des Schlauchs gedrückt. Über die Flüssigkeit wird der Ultraschall vom Ultraschallsender zum Ultraschallempfänger übertragen, ohne dass im Schallweg eine Stoßstelle vorhanden wäre. Ein solcher Ultraschallsender erfordert zwei mit Flüssigkeit gefüllte Kammern. Er ist teuer und gegen mechanische Beschädigung empfindlich.

Ein anderer Ultraschallsensor ist im US-Patent 4 418 565 beschrieben. Dieser Ultraschallsensor weist zwischen Sender und Empfänger zwei Silikonkörper auf, die von entgegengesetzten Seiten her gegen den Schlauch drücken und diesen deformieren. Die Vorrichtung eignet sich jeweils nur für einen bestimmten Schlauchdurchmesser.

Der Erfindung liegt die Aufgabe zugrunde, einen Ultraschallsensor zu schaffen, der kostengünstig herstellbar ist und eine hohe Signalausbeute liefert.

Die Lösung dieser Aufgabe erfolgt erfindungsgemäß mit den Merkmalen des Anspruchs 1. Hiernach weist die den Schlauch aufnehmende Aufnahmevorrichtung starre konkave Formungsbereiche auf, welche einen Formungskanal von im wesentlichen ovalen Querschnitt begrenzen. Der Schlauch wird teilweise flachgedrückt, wobei er sich mit seinen Biegebereichen vollflächig gegen die Formungsbereiche der Aufnahmevorrichtung legt. Somit steht an den Biegebereichen eine luftfreie Anlage des Schlauchs an den Formungsbereichen. Da die Schallgeschwindigkeit in dem Schlauchmaterial in der Regel größer ist als in der Flüssigkeit, die sich im Schlauch befindet, entsteht an der Schlauchkrümmung an der Grenzfläche zwischen Schlauch und der darin enthaltenen Flüssigkeit eine akustische Linse, durch die der Schall bzw. Ultraschall auf einen Brennpunkt gebündelt wird. Hinter dem Brennpunkt divergieren die Schallwellen wieder und von der gegenüberliegenden akustischen Linse werden sie wieder parallelgerichtet. Durch die Linsenwirkung des teilweise flachgedrückten Schlauchs werden Streuverluste der Schallenergie vermieden. Außerdem wird erreicht, das sämtliche Schallwellen auf dem Weg vom Ultraschallsender zum Ultraschallempfänger gleiche Laufzeiten haben und somit phasengleich am Empfänger eintreffen.

Der Ultraschallsensor ist einfach herstellbar. Er benötigt keine Flüssigkeitskammern zur Kopplung des Ultraschalls an die Schlauch-Aufnahmevorrichtung und gewährleistet eine hohe Signalausbeute.

Gemäß einer bevorzugten Ausgestaltung der Erfindung ist der Umfang des Formungskanals kleiner als derjenige des unverformten Schlauchs. Dadurch wird erreicht, dass das elastische Schlauchmaterial in seinem Umfang gestaucht wird. Die Stauchung bewirkt eine Kraft, die die Schlauchwandung gegen die Formungsbereiche presst. Dadurch wird auf einfache Weise gewährleistet, dass keine Lufteinschlüsse im Laufweg des Ultraschallsignals vorhanden sind.

Gemäß einer bevorzugten Ausgestaltung der Erfindung ist mindestens ein quer zu dem Schlauch bewegbarer Stempel vorgesehen, der den Schlauch deformierend in feste flächige Anlage an die Formungsbereiche drückt. Hierdurch wird die angestrebte Stauchung des Schlauchumfangs bewirkt. Generell reicht es aus, wenn ein bewegbarer Stempel vorhanden ist. Es können aber auch zwei gegenläufig zueinander bewegbare Stempel vorgesehen sein, die beim Schließen des Formungskanals auf einander zu bewegt werden, um eine Querexpansion des Schlauchs und damit eine feste vollflächige Anlage an die Formungsbereiche des Formungskanals zu bewirken. Der Ultraschallsender und der Ultraschallempfänger haben vorzugsweise einen Abstand zur Schlauchinnenwand, der einem vielfachen von $(2n-1) \cdot \lambda/4$ entspricht, wobei $\lambda$ die Wellenlänge der Ultraschallsignale in dem betreffenden Medium angibt und $n$ eine ganze Zahl ist. Bei diesem Abstand ist eine akustische Anpassung erreicht, bei der die relative Schalldurchlässigkeit ihr Maximum erreicht.

Gemäß einer speziellen Ausgestaltung der Erfindung ist eine Einrichtung vorgesehen, die aus dem Signalpegel des Empfangssignals bei blasenfreier Übertragung eine Kenngröße des Schlauchmaterials ermittelt. Dieser Aspekt der Erfindung benutzt die Erkenntnis, dass unterschiedliche Schlauchmaterialien und Schlauchwandstärken zu einer Verschiebung des Brennpunktes und/oder zu einer akustischen Fehlanpassung führen. Hieraus resultieren Signalabschwächungen, die einen Rückschluss auf das verwendete Schlauchmaterial zulassen. Dieser Parameter ist für medizinische Pumpen interessant, da das verwendete Schlauchmaterial erheblichen Einfluss auf die Fördercharakteristik hat. Der Ultraschallsensor kann also zusätzlich dazu benutzt werden, das Material oder die Beschaffenheit eines eingelegten Schlauchs zu analysieren.

Im folgenden wird unter Bezugnahme auf die Zeichnungen ein Ausführungsbeispiel der Erfindung näher erläutert.

Es zeigen:

Fig. 1    eine schematische Darstellung des Ultraschallsensors, und

Fig. 2    eine Darstellung der geometrischen Verhältnisse bei dem Ultraschallsensor.

Der Ultraschallsensor weist ein Gehäuse 10 auf, das einen Ultraschallsender 11 und einen Ultraschallempfänger 12 enthält. Der Ultraschallsender 11 und der Ultraschallempfänger 12 enthalten jeweils einen Piezokristall. Der Piezokristall des Ultraschallsenders 11 wird durch eine (nicht dargestellte) elektrische Schwingschaltung angeregt, so dass er Ultraschallsignale aussendet. Die Ultraschallsignale werden von dem Ultraschallempfänger 12 empfangen und in elektrische Signale umgewandelt.

Zwischen dem Ultraschallsender 11 und dem Ultraschallempfänger 12 befindet sich eine Aufnahmevorrichtung 13 zur Aufnahme eines Schlauchs 14. Die Aufnahmevorrichtung 13 weist starre konkave Formungsbereiche 15,16 auf, die in der jeweiligen Wand 17 des Gehäuses 10 ausgeformt sind. Die Formungsbereiche 15,16 liegen einander gegenüber. Sie sind Bestandteil eines Formungskanals 18, in dem der Schlauch 14 zu einer Ellipse zwangsgeformt wird.

Das Innere des Gehäuses 10 zwischen dem Ultraschallsender 11 und der Wand 17 ist mit einem Kunstharz 19 gefüllt, beispielsweise einem Epoxidharz. Das Gehäuse 10 besteht hier aus PVC.

Die Schallgeschwindigkeiten in der Wand 17 und im Kunststoff 19 sind etwa gleich und betragen 2500 m/sec. Die Schallgeschwindigkeit im Material des Schlauchs 14 ist etwa gleich groß wie diejenige in der Wand 17 und im Kunststoff 19 und beträgt hier 2530 m/sec. Die Schallgeschwindigkeit in der Flüssigkeit, die durch den Schlauch 14 hindurchfließt, liegt bei 1400 m/sec.

Der Ultraschallsender und der Ultraschallempfänger sind in unterschiedlichen Gehäuseteilen 10a,10b untergebracht, zwischen denen sich ein Spalt 20 befindet, der von den Wänden 17 begrenzt wird. In dem Spalt 20 sind zwei Stempel 21,22 angeordnet, die von entgegengesetzten Seiten her gegen den Schlauch 14 drücken können und den Schlauch zu einer Ellipse verformen, deren Biegebereiche gegen die Formungsbereiche 15,16 gedrückt werden. Die Stempel 21 und 22 haben jeweils eine dünne oder konkave Anlagefläche 23.

Der Schlauch 14 wird in den Führungskanal 18 eingelegt, während sich die Stempel 21 und 22 im zurückgezogenen Zustand befinden. Dann werden die Stempel 21 und 22 gegeneinander bewegt, wobei sie den Schlauch 14 stauchen und fest gegen die Formungsbereiche 15,16 drücken. In diesem Zustand erfolgt das Hindurchleiten von Ultraschall durch das Schlauchlumen.

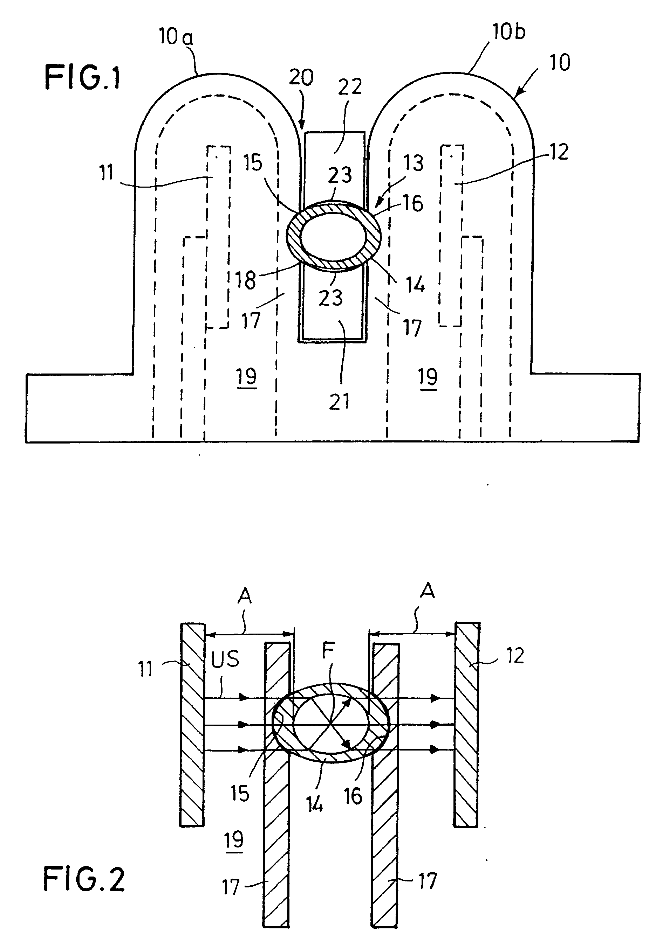

Fig. 2 zeigt den Verlauf des Ultraschalls US, der von dem Ultraschallsender 11 ausgesandt wird. Der Ultraschall verläuft in parallelen Strahlen durch den Kunststoff 19, die Wand 17 und den Schlauch 14. Bei Erreichen der Grenzfläche im Schlauchinnern werden die Ultraschallstrahlen gebeugt und auf einen Brennpunkt f fokussiert. Hinter dem Brennpunkt divergieren die Ultraschallstrahlen wieder, um an der inneren Grenzfläche des Schlauchs parallelgerichtet zu werden und dann auf den Ultraschallempfänger 12 zu treffen. Obwohl die dargestellten drei Ultraschallstrahlen unterschiedliche geometrische Längen durchlaufen, treffen sie phasengleich am Ultraschallempfänger 12 ein. Es finden daher keine phasenbedingten Auslöschungen statt.

Die Brennweite f der durch die gekrümmte Grenzfläche zwischen Schlauchinnenwand und Flüssigkeit gebildeten akustischen Linse beträgt

$$f = r/ (1-c2/c1),$$

wobei r der Krümmungsradius des Schlauchs, c1 die Schallgeschwindigkeit in der Flüssigkeit und c2 die Schallgeschwindigkeit im Schlauchmaterial is.

Der Krümmungsradius der Formungsbereiche 15,16 ist so gewählt, dass parallele Schallwellen beim Durchlaufen der ersten Grenzfläche zwischen Schlauch und Flüssigkeit durch den Mittelpunkt des Schlauchs gebrochen werden und beim Durchlaufen der zweiten Grenzfläche wieder in parallele Schallwellen umgeformt werden. Durch diese Bündelung der Schallenergie in den Schlauch und auf den Empfänger wird das Empfangssignal so verstärkt, dass das erzeugte elektrische Signal mit Dioden gleichgerichtet werden kann ohne dass eine Signalverstärkung erforderlich wäre. Da die Schallwellen beim Durchlaufen der zweiten Grenzfläche nahezu senkrecht auftreffen und wieder in parallele Schallwellen umgeformt werden, ist die Entstehung von Reflektionen und Interferenzen verhindert.

Der Abstand A zwischen Ultraschallsender und der Grenzfläche sollte ein ungeradzahliges Vielfaches von $\lambda/4$ sein, also $(2n - 1) \cdot \lambda/4$ betragen, wobei n eine ganze Zahl ist. $\lambda$ ist die Schallgeschwindigkeit in dem Kunststoff 19 bzw. der Wand 17.

**Patentansprüche**

1.   Ultraschallsensor zum Detektieren von Gasblasen in einem Schlauch (14) mit einem Ultraschallsender (11) und einem Ultraschallempfänger (12), die auf entgegengesetzten Seiten einer Aufnahmevorrichtung (13) für den Schlauch (14) angeordnet sind, **dadurch gekennzeichnet,**
**dass** die Aufnahmevorrichtung (13) starre konkave Formungsbereiche (15,16) aufweist, welche einen Formungskanal (18) von im wesentlichen ovalen Querschnitt begrenzen.

**2.** Ultraschallsensor nach Anspruch 1, **dadurch gekennzeichnet, dass** der Umfang des Formungskanals (18) kleiner ist als derjenige des unverformten Schlauchs (14).

**3.** Ultraschallsensor nach Anspruch 1 oder 2, **dadurch gekennzeichnet, dass** mindestens ein quer zu dem Schlauch (14) bewegbarer Stempel (21,22) vorgesehen ist, der den Schlauch (14) deformierend in feste flächige Anlage an die Formungsbereiche (15,16) drückt.

**4.** Ultraschallsensor nach 3, **dadurch gekennzeichnet, dass** mindestens ein Stempel (21,22) eine konkave Anlagefläche (23) für den Schlauch (14) aufweist, deren Radius größer ist als derjenige der Formungsbereiche (15,16).

**5.** Ultraschallsensor nach einem der Ansprüche 1-4, **dadurch gekennzeichnet, dass** zwischen dem Ultraschallsender (11) und dem einen Formungsbereich (15) und zwischen dem Ultraschallempfänger (12) und dem anderen Formungsbereich (16) eine Feststoffmasse (19) aus einem Material angeordnet ist, in dem die Laufgeschwindigkeit von Ultraschall etwa gleich derjenigen im Material des Schlauchs (14) ist.

**6.** Ultraschallsensor nach einem der Ansprüche 1-5, **dadurch gekennzeichnet, dass** der Abstand zwischen Ultraschallsender (11) und/oder Ultraschallempfänger (12) einerseits und der Schlauchinnenwand andererseits ein ungeradzahliges Vielfaches von $\lambda/4$ beträgt, wobei $\lambda$ die Wellenlänge der Ultraschallschwingung ist.

**7.** Ultraschallsensor nach einem der Ansprüche 1-6, **dadurch gekennzeichnet, dass** eine Einrichtung vorgesehen ist, die aus dem Signalpegel der Empfangssignale des Ultraschallempfängers (12) bei blasenfreier Übertragung eine Kenngröße des Schlauchmaterials ermittelt.

**8.** Ultraschallsensor nach Anspruch 1, **dadurch gekennzeichnet, dass** jeder Formgebungsbereich (15,16) den Schlauch (14) derart verformt, dass die Grenzfläche zwischen dem Schlauch und der darin befindlichen Flüssigkeit eine fokussierende akustische Linse bildet.

FIG.1

10a   20   22   10b   10

11   15   13   12

23   16

18   14

17   23   17

19   21   19

A   A

US   F

11   12

15   14   16

19

17   17

FIG.2

**Europäisches Patentamt**

## EUROPÄISCHER RECHERCHENBERICHT

Nummer der Anmeldung

EP 02 00 0685

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 4 668 945 A (FACCHINI MAURO ET AL) 26. Mai 1987 (1987-05-26) * Spalte 2, Zeile 28 - Spalte 4, Zeile 2; Abbildung 3 * | 1-8 | G01N29/02 G01N29/22 G01N29/28 A61M5/36 A61M1/36 |
| X | JP 03 107756 A (TERUMO CORP) 8. Mai 1991 (1991-05-08) * Zusammenfassung; Abbildung 1 * | 1,2 | |
| X | EP 0 643 301 A (COBE LAB) 15. März 1995 (1995-03-15) * Spalte 24, Zeile 43 - Zeile 56; Anspruch 1; Abbildung 18 * | 1 | |
| X | US 3 974 681 A (NAMERY JERRY) 17. August 1976 (1976-08-17) * Spalte 6, Zeile 45 - Spalte 8, Zeile 11; Abbildungen 2,3,6,9 * | 1,6,8 | |
| A | JP 58 041346 A (OLYMPUS KOGAKU KOGYO KK) 10. März 1983 (1983-03-10) * Zusammenfassung; Abbildung 1 * | 8 | RECHERCHIERTE SACHGEBIETE (Int.Cl.7) G01N A61M |

Der vorliegende Recherchenbericht wurde für alle Patentansprüche erstellt

| Recherchenort | Abschlußdatum der Recherche | Prüfer |
|---|---|---|
| MÜNCHEN | 15. April 2002 | Uttenthaler, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

# EP 1 225 445 A1

**ANHANG ZUM EUROPÄISCHEN RECHERCHENBERICHT**
**ÜBER DIE EUROPÄISCHE PATENTANMELDUNG NR.**

EP 02 00 0685

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-04-2002

| Im Recherchenbericht angeführtes Patentdokument | | Datum der Veröffentlichung | Mitglied(er) der Patentfamilie | | Datum der Veröffentlichung |
|---|---|---|---|---|---|
| US 4668945 | A | 26-05-1987 | CA | 1256977 A1 | 04-07-1989 |
| | | | DE | 3580651 D1 | 03-01-1991 |
| | | | DE | 8521951 U1 | 14-11-1985 |
| | | | EP | 0181272 A2 | 14-05-1986 |
| | | | JP | 61109554 A | 28-05-1986 |
| JP 03107756 | A | 08-05-1991 | KEINE | | |
| EP 0643301 | A | 15-03-1995 | US | 5394732 A | 07-03-1995 |
| | | | DE | 69429754 D1 | 14-03-2002 |
| | | | EP | 1182452 A2 | 27-02-2002 |
| | | | EP | 0643301 A1 | 15-03-1995 |
| | | | JP | 2823513 B2 | 11-11-1998 |
| | | | JP | 7163660 A | 27-06-1995 |
| US 3974681 | A | 17-08-1976 | KEINE | | |
| JP 58041346 | A | 10-03-1983 | JP | 1473490 C | 27-12-1988 |
| | | | JP | 63022546 B | 12-05-1988 |

EPO FORM P0461

Für nähere Einzelheiten zu diesem Anhang : siehe Amtsblatt des Europäischen Patentamts, Nr.12/82

7